# FASCICULE DE BREVET EUROPEEN

(11) **EP 2 456 766 B1**
(45) Date de publication et mention de la délivrance du brevet: **06.11.2013**
(21) Numéro de dépôt: 10734731.2
(22) Date de dépôt: 16.07.2010
(51) Int. Cl.: C07D 401/14, C22B 60/02

(54) **COMPOSES UTILES COMME LIGANDS DES ACTINIDES, LEUR SYNTHESE ET LEURS UTILISATIONS**
VERBINDUNGEN ZUR VERWENDUNG ALS ACTINIDLIGANDEN, IHRE SYNTHESE UND VERWENDUNG
COMPOUNDS SUITABLE FOR USE AS ACTINIDE LIGANDS, SYNTHESIS AND USES THEREOF

(30) Priorité: 21.07.2009 FR 0955069
(43) Date de publication de la demande: 30.05.2012
(73) Titulaire: Commissariat à l'Énergie Atomique et aux Énergies Alternatives, 75015 Paris (FR); Centre National de la Recherche Scientifique, 75016 Paris (FR); Université de Nantes, 44035 Nantes Cedex 1 (FR)
(72) Inventeur: MARIE, Cécile, F-84000 Avignon (FR); MIGUIRDITCHIAN, Manuel, F-84000 Avignon (FR); BISSON, Julia, F-50100 Cherbourg (FR); GUILLANEUX, Denis, F-30400 Villeneuve Les Avignon (FR); DUBREUIL, Didier, F-44710 Port Saint Pere (FR)
(74) Mandataire: Ilgart, Jean-Christophe
(86) Numéro de dépôt international: PCT/EP2010/060280
(87) Numéro de publication internationale: WO 2011/009814

(56) Documents cités:
- EP-B1- 0 850 322
- PETITJEAN A ET AL: "Dynamic devices. Shape switching and substrate binding in ion-controlled nanomechanical molecular tweezers" JOURNAL OF THE AMERICAN CHEMICAL SOCIETY, AMERICAN CHEMICAL SOCIETY, NEW YORK, USA, vol. 126, no. 21, 2 juin 2004 (2004-06-02) , pages 6637-6647, XP002523373 ISSN: 0002-7863 [extrait le 2004-05-05]

## Description

### DOMAINE TECHNIQUE

La présente invention se rapporte à de nouveaux composés qui sont utiles comme ligands des actinides.

Elle se rapporte également à la synthèse de ces composés et à leurs utilisations.

Les composés selon l'invention, qui présentent une affinité plus marquée pour les actinides que pour les lanthanides et qui sont capables d'extraire les actinides d'une solution aqueuse fortement acide comme une solution d'acide nitrique de molarité au moins égale à 2, sont notamment susceptibles d'être utilisés dans le domaine du traitement des combustibles nucléaires usés par voie hydrométallurgique, en particulier pour séparer de façon groupée l'ensemble des actinides (uranium, plutonium, neptunium, américium et/ou curium) et, notamment, des lanthanides présents dans des solutions de dissolution de combustibles nucléaires usés.

### ÉTAT DE LA TECHNIQUE ANTÉRIEURE

Pour séparer de façon groupée le plutonium, le neptunium, l'américium, le curium et éventuellement l'uranium des lanthanides présents dans une solution de dissolution d'un combustible nucléaire usé, il a été proposé récemment deux procédés qui utilisent comme extractant, un malonamide comme le *N,N*'-diméthyl-*N,N*'-dioctylhexyl-éthoxymalonamide (ou DMDOHEMA) ou un diglycolamide comme le *N,N,N*',*N*'-tétraoctyl-3-oxapentanediamide (ou TODGA).

Ces procédés sont respectivement décrits dans les demandes internationales PCT publiées sous les n° WO 2007/118904 (référence **[1]**) et WO 2008/099807 (référence **[2]**).

Il se trouve que les extractants présentant des atomes d'oxygène donneurs tels que les malonamides et les diglycolamides ne permettent pas d'extraire, d'une solution aqueuse acide contenant à la fois des actinides et des lanthanides, les actinides sans extraire en même temps les lanthanides.

De ce fait, les procédés décrits dans les références précitées comprennent tout d'abord une étape visant à co-extraire les actinides et les lanthanides de la solution aqueuse dans laquelle ils se trouvent, au moyen d'une phase organique qui contient le malonamide ou le diglycolamide. Cette étape de co-extraction est suivie d'une étape visant à désextraire sélectivement les actinides de la phase organique, que l'on réalise au moyen d'une phase aqueuse faiblement acide, c'est-à-dire d'un pH compris entre 2 et 3, et contenant un agent complexant, par exemple un acide polyaminocarboxylique. Les lanthanides sont alors retenus dans la phase organique soit grâce à la présence dans cette phase organique d'un extractant acide du type acide phosphorique (référence **[1]**) soit grâce à la présence dans la phase aqueuse faiblement acide d'ions nitrate (référence **[2]**). Vient ensuite une étape visant à désextraire les lanthanides de la phase organique pour, d'une part, récupérer ces lanthanides dans une phase aqueuse propre à être soumise ultérieurement aux opérations de vitrification, et, d'autre part, épurer la phase organique en radioéléments en vue de sa réutilisation.

Or, dans la perspective d'un développement de nouveaux procédés de traitement des combustibles nucléaires usés, il serait souhaitable de disposer d'extractants permettant d'isoler de façon groupée tous les actinides présents dans des solutions de dissolution de combustibles nucléaires usés. Les procédés de traitement des combustibles nucléaires usés s'en trouveraient notablement simplifiés et donc moins coûteux à mettre en oeuvre.

On connaît des composés qui présentent une affinité plus grande pour les actinides et, en particulier, pour les actinides(III) que pour les lanthanides.

I1 s'agit de composés polyaromatiques azotés comme la 2, 2': 6', 2"-terpyridine et certains de ses dérivés alkylés, la 2,4,6-tri(2-pyridinyl)-1,3,5-triazine (ou TPTZ), la 2,6-bis(pyridin-2-yl)-4-amino-1,3,5-triazine (ou ADPTZ) et les 2,6-bis(1,2,4-triazinyl)pyridines, de picolinamides, de dipicolinamides et de bipyridines à substitutions amide. La TPTZ ainsi que d'autres triazines substituées par des groupes pyridinyles sont notamment décrites dans EP 0 850 322 B1.

Toutefois, aucun de ces composés n'apparaît susceptible d'être utilisé dans un procédé industriel qui viserait à séparer de façon groupée l'ensemble des actinides présents dans des solutions de dissolution de combustibles nucléaires usés des lanthanides également présents dans ces solutions, soit parce qu'ils sont tout simplement incapables d'extraire seuls les actinides d'une phase aqueuse fortement acide (ce qui est, par exemple, le cas de la 2,2':6',2"-terpyridine et de ses dérivés alkylés, de la TPTZ, de l'ADPTZ et des picolinamides qui ne sont capables d'extraire qu'à faible acidité et en mélange synergique avec un autre extractant, typiquement, l'acide α-bromodécanoïque), soit parce qu'ils présentent une capacité de charge trop faible (ce qui est, par exemple, le cas des 2,6-bis(1,2,4-triazinyl)pyridines), soit encore parce qu'ils nécessitent d'être en solution dans un diluant polaire, halogéné et toxique comme le chloroforme ou le méta-nitrotrifluorotoluène, et donc difficilement utilisables dans un procédé industriel (ce qui est, par exemple, le cas des dipicolinamides).

Les Inventeurs se sont donc fixé pour but de fournir de nouveaux composés qui non seulement présentent une affinité plus grande pour les actinides que pour les lanthanides mais soient de plus capables d'extraire d'une solution aqueuse fortement acide l'ensemble des actinides présents dans cette solution de sorte que ces composés permettent d'isoler de façon groupée tous les actinides présents à différents degrés d'oxydation dans une solution de dissolution de combustibles nucléaires usés.

Ils se sont, de plus, fixé pour but que les composés en question puissent être employés en solution dans un diluant apte à être utilisé dans un procédé industriel de traitement de combustibles nucléaires usés.

### EXPOSÉ DE L'INVENTION

Ces buts et d'autres encore sont atteints par l'invention qui propose, en premier lieu, un composé qui répond à la formule générale (I) ci-après : 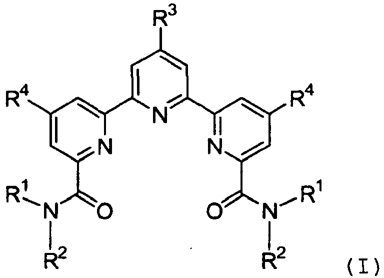 dans laquelle :
- R¹ et R², identiques ou différents, représentent un atome d'hydrogène, un groupe hydrocarboné, saturé ou insaturé, linéaire ou ramifié, en C₁ à C₁₂, un groupe phényle, un groupe benzyle, un groupe biphényle ou un groupe tolyle ;
- R³ représente un atome d'hydrogène, un groupe hydrocarboné, saturé ou insaturé, linéaire ou ramifié, en C₁ à C₁₂, un groupe phényle, un groupe tolyle ou un groupe alcoxy, linéaire ou ramifié, en C₁ à C₁₂ ; tandis que
- R⁴ représente un atome d'hydrogène, un groupe hydrocarboné, saturé ou insaturé, linéaire ou ramifié, en C₁ à C₁₂, un groupe phényle ou un groupe tolyle.

Ainsi, le composé selon l'invention a la particularité de comprendre à la fois un motif terpyridine et deux groupes amides, ces derniers étant respectivement situés sur l'un des cycles pyridine latéraux du motif terpyridine.

Dans ce qui précède et ce qui suit, on entend par « groupe hydrocarboné, saturé ou insaturé, linéaire ou ramifié, en C₁ à C₁₂ », tout groupe alkyle, à chaîne linéaire ou ramifiée, qui comprend au moins 1 atome de carbone mais pas plus de 12 atomes de carbone et tout groupe alcényle ou alcynyle, à chaîne linéaire ou ramifiée, qui comprend au moins 2 atomes de carbone et pas plus de 12 atomes de carbone.

De tels groupes hydrocarbonés sont, par exemple, les groupes méthyle, éthyle, *n*-propyle, isopropyle, butyle comme le *n*-butyle, le *sec*-butyle ou l'isobutyle, pentyle comme le *n*-pentyle, le *sec*-pentyle ou l'isopentyle, hexyle comme le *n*-hexyle ou l'isohexyle, octyle comme le *n*-octyle ou l'isooctyle, décyle comme le *n*-décyle ou l'isodécyle, dodécyle, éthylényle, propylényle, butényle, pentényle, hexényle, méthyl-pentényle, buta-1,3-diényle, octényle, décényle, dodécényle, éthynyle, propynyle, butynyle, pentynyle, hexynyle, octynyle, décynyle, dodécynyle, etc.

Par ailleurs, on entend par « groupe alcoxy, linéaire ou ramifié, en C₁ à C₁₂ », tout groupe O-alkyle dans lequel le groupe alkyle est à chaîne linéaire ou ramifiée et comprend de 1 à 12 atomes de carbone.

Un tel groupe alcoxy est, par exemple, un groupe méthoxy, éthoxy, *n*-propoxy, isopropoxy, butoxy comme le *n*-butoxy, le sec-butoxy ou l'isobutoxy, pentoxy comme le *n*-pentoxy, le *sec*-pentoxy ou l'isopentoxy, hexyloxy comme le *n*-hexyloxy ou l'isohexyloxy, octoxy comme le *n*-octoxy ou l'isooctoxy, décyloxy comme le *n*-décyloxy ou l'isodécyloxy, dodécyloxy, etc.

Conformément à l'invention, le composé répond, de préférence, à la formule générale (I) dans laquelle :
- R¹ et R², identiques ou différents, représentent un atome d'hydrogène, une chaîne alkyle, linéaire ou ramifiée, en C₁ à C₁₂ ou un groupe phényle ;
- R³ représente un atome d'hydrogène ou un groupe alkyle ou alcoxy, linéaire ou ramifié, en C₁ à C₁₂ ; tandis que
- R⁴ représente un atome d'hydrogène ou un groupe alkyle, linéaire ou ramifiée, en C₁ à C₁₂.

Plus encore, on préfère que, dans la formule générale (I), R³ et R⁴ représentent un atome d'hydrogène pour la simple raison que la synthèse de terpyridines ne comportant aucun autre substituant que les deux groupes amides portés par les cycles pyridine latéraux du motif terpyridine est plus simple à mettre en oeuvre quel celle de terpyridines davantage substituées.

Egalement, on préfère que, dans la formule générale (I), R¹ et R² soient identiques, auquel cas ils représentent avantageusement une chaîne alkyle, linéaire ou ramifiée, en C₁ à C₁₂ et, mieux encore une chaîne à nombre pair d'atomes de carbone, c'est-à-dire en C₂, en C₄, en C₅, en C₈, en C₁₀ ou en C₁₂.

De tels composés sont, par exemple :
- le *N,N,N*',*N*'-tétraéthyl-6,6"-(2,2':6',2"-terpyridine)diamide, qui répond à la formule générale (I) dans laquelle R¹ = R²= C₂H₅ tandis que R³ = R⁴ = H ;
- le *N,N,N',N'*-tétrabutyl-6, 6"-(2,2':6',2"-terpyridine)diamide, qui répond à la formule générale (I) dans laquelle R¹ = R² = C₄H₉ tandis que R³ = R⁴ = H ; et
- le *N,N,N',N'*-tétraoctyl-6,6"-(2,2':6',2"-terpyridine)diamide, qui correspond au composé de formule générale (I) dans laquelle R¹ = R² = C₈H₁₇ tandis que R³ = R⁴ = H.

Toutefois, des composés de formule générale (I) dans laquelle R¹ et R² n'ont pas la même signification comme, par exemple, le *N,N'*-diéthyl-*N,N'-*diphényl-6, 6"- (2, 2': 6', 2"-terpyridine) diamide, qui répond à la formule générale (I) dans laquelle R¹ représente un groupe phényle tandis que R² représente un groupe éthyle, peuvent également très bien convenir.

L'invention a également pour objet un procédé de synthèse d'un composé tel que précédemment défini, qui comprend la réaction d'un composé de formule générale (II) ci-après : 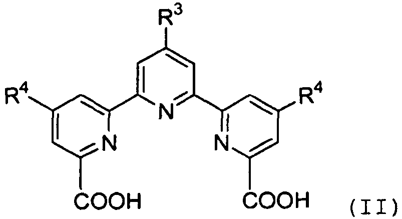 dans laquelle R³ et R⁴, identiques ou différents, ont la même signification que dans la formule générale (I), avec une amine de formule HNR¹R² dans laquelle R¹ et R², identiques ou différents, ont la même signification que dans la formule générale (I), en présence d'un agent de couplage peptidique et d'un catalyseur de couplage peptidique.

Conformément à l'invention, cette réaction peut être réalisée en utilisant tout agent de couplage et tout catalyseur de couplage dont l'utilisation a été proposée pour la synthèse peptidique. Ainsi, on peut notamment utiliser le chlorhydrate de 1-éthyl-3-[3-diméthylaminopropyl]carbodiimide comme agent de couplage et le *N*-hydroxybenzotriazole comme catalyseur de couplage, en solution dans un solvant organique dipolaire et aprotique comme, par exemple, le diméthyl-formamide anhydre.

Le composé de formule générale (II) peut, être obtenu par hydrolyse du 2,2':6',2"-terpyridine-6, 6"-dicarbonitrile correspondant en milieu basique hydroalcoolique et par traitement du produit résultant de l'hydrolyse au reflux d'une solution d'acide sulfurique et d'acide acétique, comme décrit par C. Galaup et al. dans Journal of Organic Chemistry 2005, 70(6), 2274-2284 (référence **[3]**).

Le 2,2':6',2"-terpyridine-6,6"-dicarbonitrile peut être obtenu par réaction du 2,2':6',2"-terpyridine-1,1"-dioxyde correspondant avec du cyanotriméthylsilane en présence de chlorure de benzoyle, comme décrit par W. K. Fife dans Journal of Organic Chemistry 1983, 48(8), 1375-1377 (référence **[4]**), tandis que le 2,2':6',2"-terpyridine-1,1"-dioxyde peut, lui, être obtenu par oxydation de l'atome d'azote de chacun des cycles pyridine latéraux de la 2,2':6',2"-terpyridine correspondante par l'acide *méta*-chloroperbenzoïque, comme décrit par R. P. Thummel et Y. Jahng dans Journal of Organic Chemistry 1985, 50(19), 3635-3636 (référence **[5]**).

Si ladite 2,2':6',2"-terpyridine est substituée en positions 4, 4' et 4", c'est-à-dire que R³ et R⁴ sont différents d'un atome d'hydrogène, et si R³ est différent d'un groupe alcoxy, ou si ladite 2,2':6',2"-terpyridine est substituée en position 4' uniquement, c'est-à-dire que R³ est différent d'un atome d'hydrogène mais que R⁴ est un atome d'hydrogène, et si R³ est différent d'un groupement alcoxy, alors celle-ci peut être obtenue par la méthode décrite par F. H. Case et T. J. Kasper dans Journal of the American Chemical Society 1956, 78, 5842-5844 (référence **[6]**), c'est-à-dire par réaction d'une 2-acétylpyridine fonctionnalisée en position 4 par le groupe R⁴ souhaité avec l'aldéhyde R³CHO substitué par le groupe R³ souhaité, en présence d'acétate d'ammonium et d'ammoniaque en solution aqueuse, à température élevée.

Si ladite 2,2':6',2"-terpyridine est substituée en positions 4' par un groupe alcoxy, c'est-à-dire que R³ est un groupe alcoxy, alors celle-ci peut être obtenue par réaction de la 4'-chloro-2,2':6',2"-terpyridine fonctionnalisée en positions 4 et 4" par le groupe R⁴ souhaité avec un alcool de formule R-OH dans laquelle R représente une chaîne alkyle en C₁ à C₁₂, dans du diméthylsulfoxide et en présence d'hydroxyde de potassium, comme décrit par G. R. Newkome et al. dans Journal of the Chemical Society, Chemical Communications 1993, 925-927 (référence **[7]**).

La 4'-chloro-2,2':6',2"-terpyridine fonctionnalisée en positions 4 et 4" par le groupe R⁴ souhaité est, elle, préparée en trois étapes dans les conditions décrites par E. C. Constable et al. dans Journal of the Chemical Society, Dalton Transactions 1990, 1405-1409 (référence **[8]**). Dans ce cas, on fait tout d'abord réagir de l'acétone avec un excès de 2-pyridinyl-carboxylate d'éthyle fonctionnalisé en position 4 par le groupe R⁴ souhaité pour obtenir l'intermédiaire 1,3,5-trione correspondant qui, par réaction avec l'acétate d'ammonium, permet d'obtenir ensuite la 4'-hydroxy-2,2':6',2"-terpyridine fonctionnalisée en positions 4 et 4" par le groupe R⁴ souhaité. Cette dernière est alors chlorée avec du pentachlorure de phosphore ou de l'oxychlorure de phosphore.

Les composés selon l'invention se sont révélés présenter une affinité pour les actinides et ce, quel que soit leur degré d'oxydation : III, IV, V ou VI. De plus, cette affinité est supérieure à celle qu'ils présentent vis-à-vis des lanthanides.

Par ailleurs, les composés selon l'invention se sont révélés être capables d'extraire l'ensemble des actinides d'une phase aqueuse acide et, en particulier, d'une phase aqueuse fortement acide comme une solution d'acide nitrique de molarité supérieure ou égale à 2.

De ce fait, l'invention a encore pour objet l'utilisation d'un composé tel que précédemment défini en tant que ligand des actinides et, en particulier, pour extraire un ou des actinides d'une solution aqueuse acide par la technique d'extraction liquide-liquide.

Dans le cadre de cette utilisation, le composé peut notamment être utilisé pour séparer les actinides présents dans une solution aqueuse acide des lanthanides qui sont également présents dans cette solution.

Conformément à l'invention, la solution aqueuse acide est, de préférence, une solution d'acide nitrique de molarité au moins égale à 2.

Une telle solution aqueuse est, par exemple, une solution issue de la dissolution d'un combustible nucléaire usé dans de l'acide nitrique.

Toutefois, il peut également s'agir d'une solution aqueuse issue de la dissolution d'un combustible nucléaire usé dans de l'acide nitrique mais que l'on a préalablement débarrassée de l'uranium qu'elle contient.

Dans tous les cas, le composé est avantageusement utilisé en solution, à raison de 0,1 à 2 mole/L, dans un solvant organique, lequel est, de préférence, choisi parmi le n-octanol, le nitrobenzène, le n-dodécane et le tétrapropylène hydrogéné (ou TPH).

Typiquement, l'utilisation du composé pour séparer les actinides des lanthanides présents dans une solution aqueuse acide comprend :
- l'extraction des actinides de la solution aqueuse par mise en contact aveç une phase organique comprenant ce composé dans un diluant organique du type de ceux précités puis séparation de ladite solution aqueuse et de ladite phase organique ; et
- la désextraction des actinides présents dans la phase organique obtenue à l'issue de l'extraction par mise en contact de cette phase avec une phase aqueuse acide, de préférence de pH allant de 2 à 3.

D'autres caractéristiques et avantages de l'invention apparaîtront mieux à la lecture du complément de description qui suit, qui se rapporte à des exemples de synthèse de composés selon l'invention et de démonstration de leur aptitude à être utilisés pour extraire les actinides d'une solution aqueuse fortement acide et pour les séparer des lanthanides se trouvant également dans cette solution.

Bien entendu, ces exemples ne sont donnés qu'à titre d'illustrations de l'objet de l'invention et ne constituent en aucun cas une limitation de cet objet.

### BRÈVE DESCRIPTION DES DESSINS

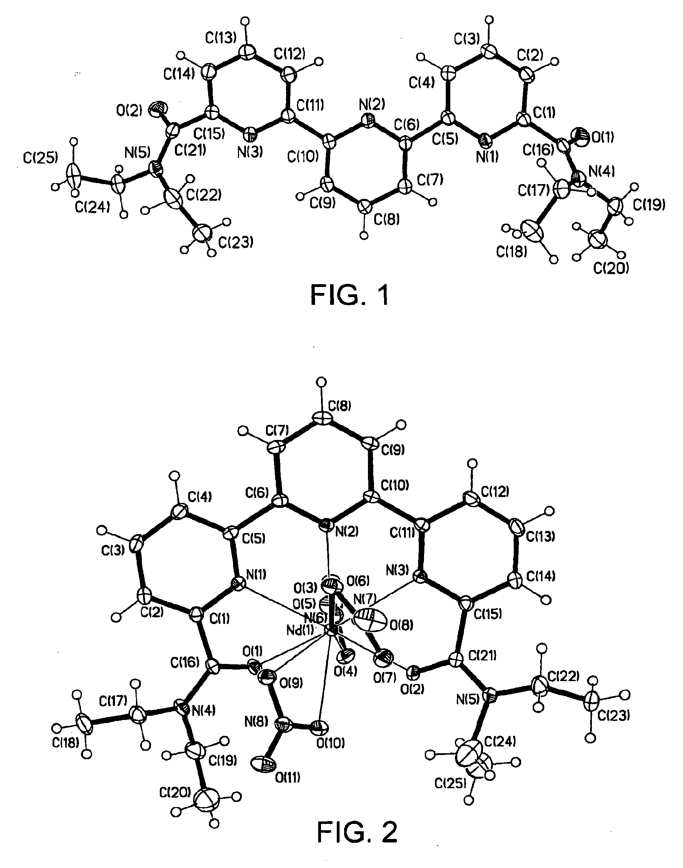

La figure 1 correspond à la représentation ORTEP de la structure cristallographique d'un premier composé selon l'invention, telle que résolue par diffraction des rayons X.
La figure 2 correspond à la représentation ORTEP de la structure cristallographique du complexe formé par le composé de la figure 1 et le néodyme(III), telle que résolue par diffraction des rayons X.

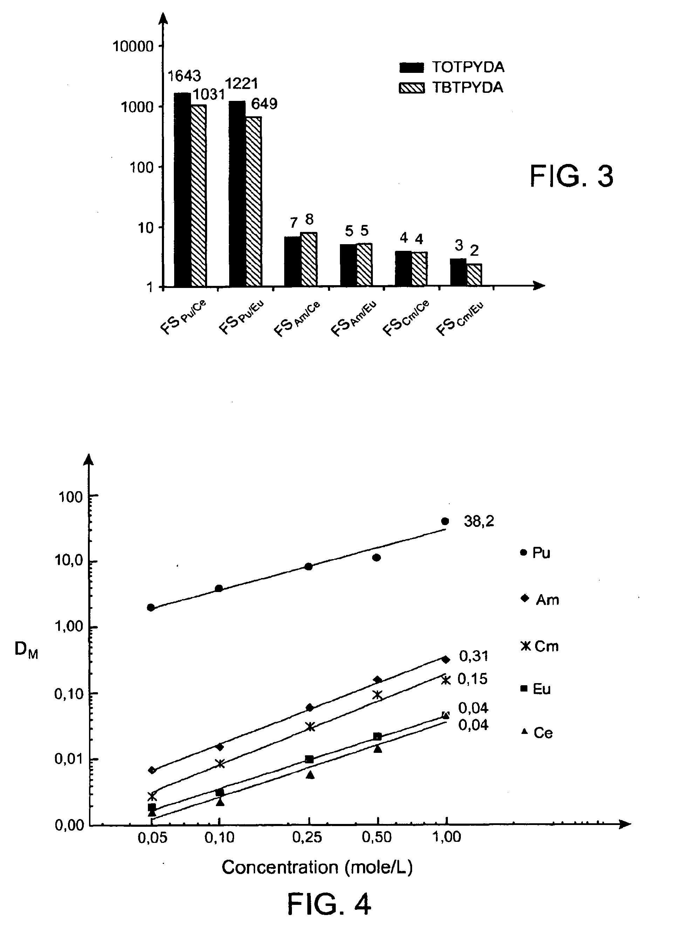

La figure 3 représente, sous la forme d'un diagramme en bâtons, les facteurs de séparation FS_{Pu/Ce}, FS_{Pu/Eu}, FS_{Am/Ce}, FS_{Am/Eu}, FS_{Cm/Ce} et FS_{Cm}/_{Eu} tels qu'obtenus lors d'extractions réalisées sur une phase aqueuse nitrique contenant du plutonium, de l'américium, du curium, de l'europium et du cérium, au moyen de deux phases organiques contenant pour la première, un deuxième composé selon l'invention (bâtons pleins) et pour la seconde, un troisième composé selon l'invention (bâtons hachurés) dans du *n*-octanol.
La figure 4 représente la variation des coefficients de distribution (D_{M}) du plutonium, de l'américium, du curium, de l'europium et du cérium tels qu'obtenus lors d'extractions réalisées sur des phases aqueuses nitriques contenant du plutonium, de l'américium, du curium, de l'europium et du cérium, au moyen de phases organiques contenant le deuxième composé selon l'invention dans du n-octanol, en fonction de la concentration de ce composé dans lesdites phases organiques.

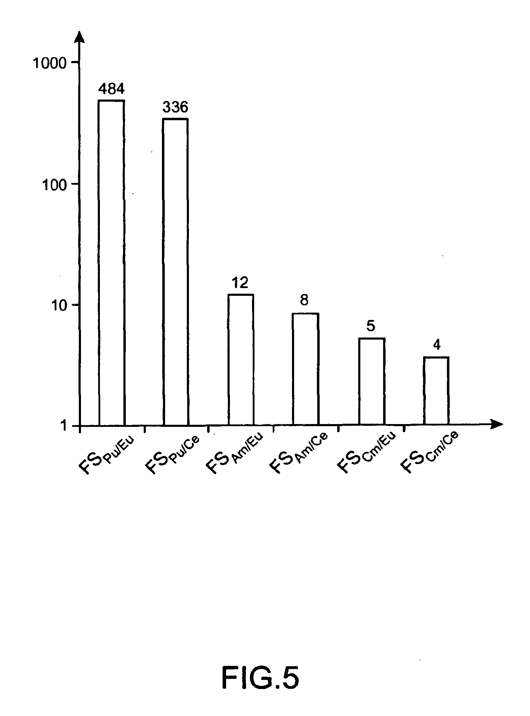

La figure 5 représente, sous la forme d'un diagramme en bâtons, les facteurs de séparation FS_{Pu/Ce}, FS_{Pu/Eu}, FS_{Am/Ce}, FS_{Am/Eu}, FS_{Cm/Ce} et FS_{Cm/Eu} tels qu'obtenus lors d'extractions réalisées sur une phase aqueuse nitrique contenant du plutonium, de l'américium, du curium, de l'europium et du cérium, au moyen d'une phase organique contenant le troisième composé selon l'invention dans du nitrobenzène.

### EXPOSÉ DÉTAILLÉ DE MODES DE RÉALISATION PARTICULIERS

### EXEMPLE 1 : SYNTHESE DE COMPOSES SELON L'INVENTION

Les composés selon l'invention sont synthétisés à partir de l'acide 2,2'6',2"-terpyridine-6,6"-dicarboxylique, noté ci-après composé 4, lequel est préalablement obtenu à partir de 1a 2, 2': 6', 2"-terpyridine, notée ci-après composé 1, selon le schéma réactionnel suivant :

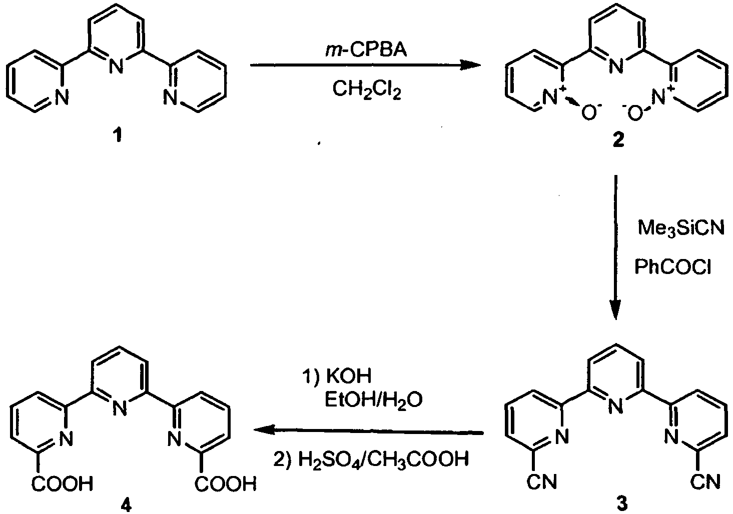

Le composé 1 est commercialement disponible.

Le composé 2 est tout d'abord obtenu par oxydation de l'atome d'azote des deux cycles pyridine latéraux du composé 1 avec 4 équivalents d'acide *méta-*chloroperbenzoïque (ou *m*-CPBA) dans les conditions décrites dans la référence **[5]** précitée (Rendement : 73%).

Puis, le composé 3 est obtenu en faisant réagir le composé 2 avec 10 équivalents de cyanotriméthylsilane (ou Me₃SiCN) en présence de 4 équivalents de chlorure de benzoyle (ou PhCOCl) dans les conditions décrites dans la référence **[4]** précitée (Rendement : 88%).

Le composé 3 est ensuite hydrolysé en milieu basique hydroalcoolique (KOH/éthanol/H₂O) avant de subir un traitement au reflux d'une solution d'acide sulfurique et d'acide acétique comme décrit dans la référence **[3]** précitée (Rendement : 96%).

### 1.1. Synthèse du N,N,N',N'-tétraéthyl-6,6"-(2,2':6',2"-terpyridine) diamide

Le composé titre, noté ci-après TETPYDA, est obtenu par réaction du composé 4 avec la N,N-diéthylamine.

Pour ce faire, 350 mg (1, 1 mmole) du composé 4 et 100 mg de *N*-hydroxybenzotriazole (ou HOBt - 0,7 mmole) sont dissous dans 3 mL de DMF anhydre. Puis, sont ajoutés à cette solution 440 mg de chorhydrate de 1-éthyl-3-[3-diméthylaminopropyl]carbodiimide (ou EDC - 2,3 mmoles) et 237 µL de *N,N*-diéthyl-amine (2,3 mmoles) et le mélange résultant est agité pendant 18 heures, à température ambiante et sous atmosphère d'argon. Après évaporation du solvant, le produit brut est repris dans du CH₂Cl₂ et lavé trois fois avec de l'eau distillée. La phase organique est ensuite séchée sur MgSO₄ et le solvant est évaporé. Le résidu obtenu est purifié par chromatographie sur gel de silice (élution par un mélange CH₂Cl₂/MeOH 95:5 en mode isocratique).

Après évaporation des solvants, on obtient 300 mg de TETPYDA (Rendement : 64%) sous la forme d'une poudre blanche qui est constituée d'un mélange de deux rotamères représentant chacun 50% en masse de ce mélange.
**Formule brute :** C₂₅H₂₉N₅O₂
**Masse molaire :** 431 g/mole
**Point de fusion :** 132°C
**RMN ¹H (300 MHz, CDCl₃) :**
rotamère 1 : δ ppm : 8.66 (dd, *³J =* 7.8, *⁴J =* 0.9, 2H, H_{pyridine}), 8.46 (d, *³J* = 7.8, 2H, H_{pyridine}), 7.97 (t, *³J* = 7.8, 2H, H_{pyridine}), 7.94 (t, *³J* = 7.8, 1H, H_{pyridine}), 7.66 (dd, *³J =* 7.8, *⁴J* = 0.9, 2H, H_{pyridine}), 3.63 (q, *³J* = 7.0, 8H, CH₂), 1.32 (t, *³J* = 7.0, 12H, CH₃)
rotamère 2 : tous les signaux coïncident avec le rotamère 1 exceptés ceux à 3.47 (q, *³J* = 7.0, 8H, CH₂), 1.27 (t, *³J* = 7.0, 12H, CH₃)
**RMN ¹³C (75 MHz, CDCl₃) :**
rotamère 1 : δ ppm : 168.5 (2C=O), 154.9 (2Cq), 154.5 (4Cq), 137.9 (2CH_{pyridine}), 137.7 (CH_{pyridine}), 123.4 (2CH_{pyridine}), 121.4 (2CH_{pyridine}) , 121.3 (2CH_{pyridine}) , 40.3 (4CH₂), 12.9 (4CH₃)
rotamère 2 : tous les signaux coïncident avec ceux du rotamère 1 exceptés les signaux à 43.3 (4CH₂), 14.5 (4CH₃)
**Spectrométrie de masse (EI), m/z (I%):** 431 (M⁺, 30%), 232 (M⁺-2CONEt₂, 82%), 72 (⁺NEt₂, 100%)
**Pureté HPLC à 210 nm :** 99,6%

La structure cristallographique du TETPYDA ainsi que celle du complexe formé par ce composé après réaction avec 1 équivalent de nitrate de néodyme(III) ont été résolues par diffraction des rayons X. Leur représentation ORTEP est illustrée sur les figures 1 et 2.

Les cristaux de TETPYDA et de son complexe avec le néodyme(III) ont été obtenus dans le méthanol par évaporation lente.

La figure 1 montre qu'à l'état cristallin, le TETPYDA en conformation *s-trans* n'est pas pré-organisé en vue d'une complexation puisque les atomes d'azote des cycles pyridine sont en position opposée les uns par rapport aux autres et les fonctions carbonyles sont orientées pratiquement perpendiculairement au plan de ces cycles, tandis que la figure 2 montre qu'après complexation, le TETPYDA est pentadenté et forme un complexe de stoechiométrie 1:1 avec le néodyme(III). Ce dernier est complexé en sphère interne à la fois par les atomes d'oxygène (durs) des groupes amides et par les atomes d'azote des cycles pyridine (plus mous selon la théorie de Pearson).

### 1.2. Synthèse du N,N,N',N'-tétrabutyl-6, 6"-(2,2':6',2"-terpyridine) diamide

Le composé titre, noté ci-après TBTPYDA, est obtenu par réaction du composé 4 avec la *N,N*-dibutylamine.

Pour ce faire, 1,4 g (4,3 mmoles) du composé 4 et 383 mg d'HOBt (2,5 mmoles) sont dissous dans 13 mL de DMF anhydre. Puis, sont ajoutés à cette solution 1,7 g d'EDC (9,0 mmoles) et 1,5 mL de *N,N*-dibutylamine (9,0 mmoles) et le mélange résultant est agité pendant 18 heures, à température ambiante et sous atmosphère d'argon. Après évaporation du solvant, le produit brut est repris dans du CH₂Cl₂ et lavé avec une solution aqueuse d'HCl 1 N puis avec une solution aqueuse de bicarbonate de sodium à 5%. La phase organique est ensuite séchée sur MgSO₄ et le solvant est évaporé. Le résidu obtenu est purifié par chromatographie sur gel de silice (élution par un mélange CH₂Cl₂/acétate d'éthyle en mode gradient d'élution) et recristallisé dans l'éther de pétrole.

On obtient ainsi 970 mg de TBTPYDA (Rendement : 42%) sous la forme d'une poudre blanche qui est constituée d'un mélange de deux rotamères représentant chacun 50% en masse de ce mélange.
**Formule brute :** C₃₃H₄₅N₅O₂
**Masse molaire :** 543 g/mole
**Point de fusion :** 105°C
**RMN ¹H (400 MHz, CDCl₃) :**
rotamère 1 : δ ppm : 8.67 (d, *³J* = 8.1, 2H, H_{pyridine}), 8.45 (d, *³J* = 8.1, 2H, H_{pyridine}), 8.02-7.85 (m, 3H, Hpyridine), 7.64 (d, *³J* = 7.5, 2H, H_{pyridine}), 3.56 (t, *³J* = 7.5, 8H, CH₂), 1.81-1.59 (m, 8H, CH₂), 1.54-1.36 (m, 8H, CH₂), 1.01 (t, *³J* = 7.2, 12H, CH₃)
rotamère 2 : tous les signaux coïncident avec ceux du rotamère 1 exceptés les signaux à 3.40 (t, *³J* = 7.5, 8H, CH₂), 1.81-1.59 (m, 8H, CH₂), 1.21-1.06 (m, 8H, CH₂) et 0.77 (t, *³J* = 7.2, 12H, CH₃)
**Spectrométrie de masse (ESI), m/z (I%) :** 544 (MH⁺, 52%), 566 (MNa⁺, 36%)
**Pureté HPLC à 210 nm :** 99,9%

### 1.3. Synthèse du N,N,N',N'-tétraoctyl-6,6"-(2,2':6',2"-terpyridine) diamide

Le composé titre, noté ci-après TOTPYDA, est obtenu par réaction du composé 4 avec la *N,N*-dioctylamine.

Pour ce faire, 320 mg (1,0 mmole) du composé 4 et 92 mg d'HOBt (0,6 mmole) sont dissous dans 3 mL de DMF anhydre. Puis, sont ajoutés à cette solution 400 mg d'EDC (2,1 mmoles) et 642 µL de *N,N*-dioctylamine (2,1 mmoles) et le mélange résultant est agité pendant 18 heures, à température ambiante et sous atmosphère d'argon. Après évaporation du solvant, le produit brut est repris dans du CH₂Cl₂ et lavé trois fois avec de l'eau distillée. La phase organique est ensuite séchée sur MgSO₄ et le solvant est évaporé. Le résidu obtenu est purifié par chromatographie sur gel de silice (élution par de l'éther de pétrole, puis un mélange éther de pétrole/acétate d'éthyle 80:20 en mode isocratique).

Après évaporation des solvants, on obtient 565 mg de TOTPYDA (Rendement : 74%) sous la forme d'une huile jaune qui est constituée d'un mélange de deux rotamères représentant chacun 50% en masse de ce mélange.
**Formule brute :** C₄₉H₇₇N₅O₂
**Masse molaire :** 768 g/mole
**RMN ¹H (300 MHz, CDCl₃) :**
rotamère 1 : δ ppm : 8.66 (dd, *³J* = 7.8, *⁴J* = 0.9, 2H, H_{pyridine}), 8.44 (d, *³J* = 7.8, 2H, H_{pyridine}), 7.94 (t, *³J* =7.8, 2H, H_{pyridine}), 7.91 (t, *³J* = 7.8, 1H, H_{pyridine}), 7. 62 (dd, *³J* = 7.8, *⁴J* = 0.9, 2H, H_{pyridine}), 3.54 (t, *³J* = 7.8, 8H, CH₂), 1.73-1.68 (m, 8H, CH₂), 1.38-1.12 (m, 40H, CH₂), 0.90 (t, *³J* = 6.6, 12H, CH₃)
rotamère 2 : tous les signaux coïncident avec ceux du rotamère 1 exceptés les signaux à 3.39 (t, *³J* = 7.8, 8H, CH₂), 1.12 (m, 40H, CH₂) et 0.79 (t, *³J* = 6.6, 12H, CH₃)
**RMN ¹³C (75 MHz, CDCl₃) :**
rotamère 1 : δ ppm : 168.7 (2C=O), 154.8 (2Cq), 154.7 (2Cq), 154.4 4 (2Cq), 137.7 (3CH_{pyridine}), 123.4 (2CH_{pyridine}), 121.3 (2CH_{pyridine}), 121.2 (2CH_{pyridine}), 46.0 (4CH₂), 31.8-22.5 (20CH₂), 27.6 (4CH₂), 14.0 (4CH₃)
rotamère 2 : tous les signaux coïncident avec ceux du rotamère 1 exceptés les signaux à 49.0 (4CH₂) et 14.1 (4CH₃)
**Spectrométrie de masse (CI), m/z (I%) :** 769 (MH⁺, 85%) **Pureté HPLC à 210 nm :** 99,0%

### 1.4. Synthèse du N,N'-diéthyl-N,N'-diphényl-6, 6"- (2, 2': 6', 2"-terpyridine) diamide

Le composé titre, noté ci-après DEDPTPYDA, est obtenu par réaction du composé 4 avec la *N*-éthyl-*N-*phénylamine.

Pour ce faire, 1,2 g (3,8 mmoles) du composé 4 et 355 mg d'HOBt (2,2 mmoles) sont dissous dans 11 mL de DMF anhydre. Puis, sont ajoutés à cette solution 1,5 g d'EDC (7,9 mmoles) et 994 µL de *N*-éthyl-*N*-phénylamine (7,9 mmoles) et le mélange résultant est agité pendant 18 heures, à température ambiante et sous atmosphère d'argon. Après évaporation du solvant, le produit brut est repris dans du CH₂Cl₂ et lavé avec une solution aqueuse d'acide chlorhydrique 1 N puis avec une solution aqueuse de bicarbonate de sodium à 5%. La phase organique est ensuite séchée sur MgSO₄ et le solvant est évaporé. Le résidu obtenu est ensuite purifié par chromatographie sur gel de silice (élution par un mélange CH₂Cl₂/acétate d'éthyle en mode gradient d'élution) et recristallisé dans l'éther de pétrole.

On obtient ainsi 890 mg de DEDPTPYDA (Rendement : 44%) sous la forme d'une poudre blanche qui est constituée d'un mélange de deux rotamères représentant chacun 50% en masse de ce mélange.
**Formule brute :** C₃₃H₂₉N₅O₂
**Masse molaire :** 527 g/mole
**Point de fusion :** 168°C
**RMN ¹H (400 MHz, CDCl₃) :**
δ ppm : 8.36 (d, *³J* = 4. 6, 2H, H_{pyridine}), 7.70 (m, 7H, Hpyridine), 7.17 (m, 10H, H_{phényle}), 4.06 (q, *³J* = 7.2, 4H, CH₂), 1.28 (d, *³J* = 7.2, 6H, CH₃)
**Spectrométrie de masse (ESI), m/z (1%) :** 528 (MH⁺, 28%), 550 (MNa⁺, 61%)
**Pureté HPLC à 210 nm :** 99,4%

### EXEMPLE 2 : PROPRIETES EXTRACTANTES DES COMPOSES SELON L'INVENTION

### 2.1. Mise en évidence des propriétés extractantes des composés selon l'invention

Des extractions sont réalisées en utilisant :
- comme phases organiques : des solutions comprenant soit du TOTPYDA soit du TBTPYDA à 0,10 mole/L dans du *n*-octanol ; et
- comme phases aqueuses : quatre solutions aqueuses dénommées ci-après S1, S2, S3 et S4 et comprenant respectivement :
   S1 : un mélange de ²³⁹⁻²⁴⁰Pu(IV), ²⁴⁴Cm(III), ²⁴¹Am(III), ¹⁵²Eu(III) et ¹³⁹Ce(III), tous à l'état de traces (10⁻⁵ à 10⁻⁶ mole/L), et de l'acide nitrique à 2,8 ou 2,9 moles/L ;
   S2 : du ²³⁸U(VI) à 0,01 mole/L et de l'acide nitrique à 2,6 moles/L ;
   S3 : du ²³⁷Np(V) à 0,01 mole/L, 6% de ²³⁷Np(VI) rapportés à la masse totale de ²³⁷Np, et de l'acide nitrique à 3,0 moles/L ;
   S4 : du ²³⁷Np(VI) à 0,01 mole/L et de l'acide nitrique à 3,0 moles/L d'acide nitrique.

Chacune des phases aqueuses S1 à S4 est mise en contact, dans un tube, avec l'une des phases organiques, à raison de 1 volume de phase aqueuse pour 1 volume de phase organique, et les phases ainsi mises en contact sont laissées sous agitation mécanique pendant une heure à une température constante de 25°C.

Après quoi, les phases aqueuse et organique sont séparées l'une de l'autre et les activités ou les concentrations des différents éléments métalliques dans ces phases sont déterminées par spectrométrie α, spectrométrie γ ou par fluorescence X selon le cas.

Le tableau I ci-après présente, pour chaque extraction, les coefficients de distribution obtenus à partir des activités ou des concentrations ainsi déterminées, tandis que la figure 3 présente, pour les extractions réalisées sur la phase aqueuse S1, les facteurs de séparation FS_{Pu/Ce}, FS_{Pu/Eu}, FS_{Am/Ce}, FS_{Am/Eu}, FS_{Cm/Ce} et FS_{Cm/Eu} calculés à partir des coefficients de distribution. Sur cette figure, les bâtons pleins correspondent aux facteurs de séparation obtenus pour l'extraction réalisée avec la phase organique contenant le TOTPYDA tandis que les bâtons hachurés correspondent aux facteurs de séparation obtenus pour l'extraction réalisée avec la phase organique contenant le TBTPYDA.

On rappelle que, dans le domaine des extractions liquide-liquide, le coefficient de distribution, noté D_{M}, d'un élément M correspond au rapport, à l'équilibre, des concentrations (ou activités) de cet élément dans les phases organique et aqueuse ayant été mises en contact, et que le facteur de séparation entre deux éléments métalliques M1 et M2, noté FS_{M1/M2}, correspond à D_{M1}/D_{M2}, c'est-à-dire au rapport des coefficients de distribution des éléments métalliques M1 et M2 obtenus au cours d'une même extraction.

**TABLEAU I**

| **Phase aqueuse** | | **Elément** | **D_{M}** | |
|---|---|---|---|---|
| | | | **TOTPYDA** | **TBTPYDA** |
| **S1** | **[HNO₃] 2,9 M** | ²³⁹⁻²⁴⁰Pu (IV) | 3,8 | --- |
| | | ²⁴¹Am (III) | 1,5.10⁻² | **---** |
| | | ²⁴⁴Cm (III) | 8,7.10⁻³ | --- |
| | | ¹⁵²Eu (III) | 3,1.10⁻³ | **---** |
| | | ¹³⁹Ce (III) | 2,3.10⁻³ | --- |
| | **[HNO₃] 2,8 M** | ²³⁹⁻²⁴⁰Pu (IV) | --- | 2,4 |
| | | ²⁴¹Am (III) | --- | 1,8.10⁻² |
| | | ²⁴⁴Cm (III) | --- | 8,5.10⁻³ |
| | | ¹⁵²Eu (III) | --- | 3,7.10⁻³ |
| | | ¹³⁹Ce (III) | --- | 2,3.10⁻³ |
| **S2** | | ²³⁸U (VI) | 0, 31 | 0,46 |
| **S3** | | ²³⁷Np (V) | 0, 78 | 1, 04 |
| **S4** | | ²³⁷Np (VI) | 0, 68 | 0, 96 |

Le tableau I et la figure 3 montrent que le plutonium est particulièrement bien extrait d'une phase aqueuse acide par le TOTPYDA et le TBTPYDA puisque les coefficients de distribution D_{Pu} sont supérieurs à 2 et que les facteurs de séparation FS_{Pu/Ce} et FS_{Fu/Eu} sont supérieurs à 500.

Les éléments de degré d'oxydation VI (uranium et neptunium) sont également efficacement extraits puisqu'ils présentent des coefficients de distribution compris entre 0,3 et 1,0.

En ce qui concerne le Np(V), la phase S3 utilisée contenant 6 % de Np(VI), les coefficients de distribution obtenus ne sont pas exactement représentatifs du Np(V) seul. Ils montrent que le TOTPYDA et le TBTPYDA sont capables d'extraire d'une solution aqueuse fortement acide un mélange de Np(V) et de Np(VI), ce qui est très intéressant puisque le neptunium est présent dans les solutions de dissolution des combustibles nucléaires usés aux degrés d'oxydation V et VI. Il ne sera donc pas nécessaire de réduire le Np(VI) en Np(IV), forme souvent mieux extraite par les extractants de l'état de l'art.

Par ailleurs, comme le montre la figure 3, le TOTPYDA et le TBTPYDA présentent une plus grande affinité pour les actinides (III) que pour les lanthanides puisque les facteurs de séparation FS_{Am/Ce}, FS_{Am/Eu}, FS_{Cm/Ce} et FS_{Cm/Eu} sont tous supérieurs ou égaux à 2.

Avec une phase organique contenant du TOTPYDA ou du TBTPYDA à 0,10 mole/L dans du n-octanol, il est donc possible de séparer tous les actinides présents dans une solution aqueuse fortement acide des lanthanides également présents dans cette solution. S'il est vrai que les éléments de degré d'oxydation IV, V ou VI sont particulièrement bien extraits, la séparation des actinides (III) des lanthanides est également assurée.

### 2.2. Influence de la concentration du composé selon l'invention dans la phase organique

Des extractions sont réalisées en utilisant :
- comme phases organiques : des solutions comprenant du TOTPYDA à 0,05, 0,10, 0,25, 0,50 et 1 mole/L dans du n-octanol ; et
- comme phases aqueuses : des solutions aqueuses comprenant un mélange de ²³⁹⁻²⁴⁰Pu(IV), ²⁴⁴Cm(III), ²⁴¹Am(III), ¹⁵²Eu(III) et ¹³⁹Ce(III), tous à l'état de traces (10⁻⁵ à 10⁻⁶ mole/L), et de l'acide nitrique à 2,9 moles/L.

Comme précédemment, chaque phase organique est mise en contact, dans un tube, avec l'une des phases aqueuses, à raison de 1 volume de phase organique pour 1 volume de phase aqueuse, et les phases ainsi mises en contact sont laissées sous agitation mécanique pendant une heure à une température constante de 25°C.

Après quoi, les phases aqueuse et organique sont séparées l'une de l'autre et les activités ou les concentrations des différents éléments métalliques dans ces phases sont déterminées par spectrométrie α ou par spectrométrie γ selon le cas.

La figure 4 représente la variation des coefficients de distribution obtenus à partir des activités ou des concentrations ainsi déterminées, en fonction de la concentration en TOTPYDA de la phase organique.

L'augmentation de la concentration en TOTPYDA de la phase organique permet donc d'améliorer significativement l'extraction des actinides(III) tout en conservant une meilleure affinité pour les actinides (III) par rapport aux lanthanides (III).

De plus, les pentes des droites d'extrapolation log(D_{M}) = f(log[extractant]) sont à peu près égales à 1, ce qui confirme que les complexes formés au cours de l'extraction mettent en jeu une molécule de ligand pour un cation actinide ou lanthanide (stoechiométrie 1:1) tel que cela est observé à l'état cristallin par diffraction des rayons X.

### 2.3. Influence de la polarité du diluant de la phase organique

Une extraction est réalisée en procédant de la même façon qu'aux points 2.1 et 2.2 ci-avant mais en utilisant :
- comme phase organique : une solution comprenant du TBTPYDA à 1 mole/L dans du nitrobenzène qui est un solvant plus polaire que le n-octanol ; et
- comme phase aqueuse : une solution aqueuse comprenant un mélange de ²³⁹⁻²⁴⁰Pu(IV), ²⁴⁴Cm(III), ²⁴¹Am(III), ¹⁵²Eu(III) et ¹³⁹Ce(III), tous à l'état de traces (10⁻⁵ à 10⁻⁶ mole/L), et de l'acide nitrique à 3,1 moles/L.

Dans ces conditions, les coefficients de distribution obtenus sont nettement plus élevés que ceux obtenus en utilisant le TBTPYDA à 0,1 mole/L en solution dans du n-octanol et que ceux obtenus avec le TOTPYDA à 1 mole/L en solution dans du n-octanol puisque ces coefficients sont respectivement de 121 pour le plutonium, de 3 pour l'américium, de 1,3 pour le curium, de 0,25 pour l'europium et de 0,36 pour le cérium.

Par ailleurs, comme le montre la figure 5, qui représente les facteurs de séparation FS_{Pu/Ce}, FS_{Pu/Eu}, FS_{Am/Ce}, FS_{Am/Eu,} FS_{Cm/Ce} et FS_{Cm/Eu} calculés à partir de ces coefficients de distribution, les facteurs de séparation FS_{Am/Ce}, FS_{Am/Eu}, FS_{Cm/Ce} et FS_{Cm/Eu} sont aussi nettement plus élevés.

L'augmentation de la polarité du diluant de la phase organique permet donc d'améliorer significativement l'extraction des actinides(III) et leur séparation des lanthanides.

### REFERENCES CITEES

**[1]** Demande internationale PCT WO 2007/118904
[2] Demande internationale PCT WO 2008/049807
**[3]** C. Galaup et al., Journal of Organic Chemistry 2005, 70(6), 2274-2284
**[4]** W. K. Fife, Journal of Organic Chemistry 1983, 48 (8), 1375-1377
[5] R. P. Thummel et Y. Jahng, Journal of Organic Chemistry 1985, 50(19), 3635-3636
**[6]** F. H. Case et T. J. Kasper, Journal of the American Chemical Society 1956, 78, 5842-5844
**[7]** G. R. Newkome et al., Journal of the Chemical Society, Chemical Communications 1993, 925-927
**[8]** E. C. Constable et al., Journal of the Chemical Society, Dalton Transactions 1990, 1405-1409

## Revendications

1. Composé qui répond à la formule générale (I) ci-après : 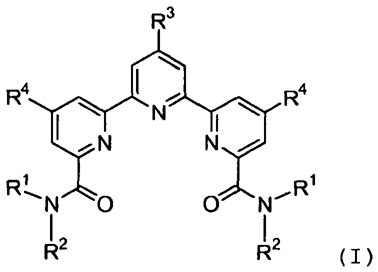 dans laquelle :
- R¹ et R², identiques ou différents, représentent un atome d'hydrogène, un groupe hydrocarboné, saturé ou insaturé, linéaire ou ramifié, en C₁ à C₁₂, un groupe phényle, un groupe benzyle, un groupe biphényle ou un groupe tolyle ;
- R³ représente un atome d'hydrogène, un groupe hydrocarboné, saturé ou insaturé, linéaire ou ramifié, en C₁ à C₁₂, un groupe phényle, un groupe tolyle ou un groupe alcoxy, linéaire ou ramifié, en C₁ à C₁₂ ; tandis que
- R⁴ représente un atome d'hydrogène, un groupe hydrocarboné, saturé ou insaturé, linéaire ou ramifié, en C₁ à C₁₂, un groupe phényle ou un groupe tolyle.

2. Composé selon la revendication 1, qui répond à la formule générale (I) dans laquelle :
- R¹ et R² identiques ou différents, représentent un atome d'hydrogène, une chaîne alkyle, linéaire ou ramifiée, en C₁ à C₁₂ ou un groupe phényle ;
- R³ représente un atome d'hydrogène ou un groupe alkyle ou alcoxy, linéaire ou ramifié, en C₁ à C₁₂ ; tandis que
- R⁴ représente un atome d'hydrogène ou un groupe alkyl, linéaire ou ramifiée, en C₁ à C₁₂.

3. Composé selon la revendication 1 ou la revendication 2, qui répond à la formule générale (I) dans laquelle R³ et R⁴ représentent un atome d'hydrogène.

4. Composé selon l'une quelconque des revendications précédentes, qui répond à la formule générale (I) dans laquelle R¹ et R² sont identiques entre eux.

5. Composé selon la revendication 4, qui répond à la formule générale (I) dans laquelle R¹ et R² représentent une chaîne alkyle, linéaire ou ramifiée, en C₁ à C₁₂ et, mieux encore, en C₂, en C₄, en C₆, en C₆, en C₁₀ ou en C₁₂.

6. Composé selon l'une quelconque des revendications précédentes, qui est choisi parmi :
- le *N,N,N',N'*-tétraéthyl-6,6"-(2,2':6',2"-terpyridine)diamide ;
- le *N,N,N'N'*-tétrabutyl-6,6"-(2,2':6',2"-terpyridine)diamide ; et
- le *N,N,N',N*'-tétraoctyl-6, 6"-(2,2':6',2"-terpyridine)diamide.

7. Procédé de synthèse d'un composé selon l'une quelconque des revendications 1 à 6, qui comprend la réaction d'un composé de formule générale (II) ci-après : 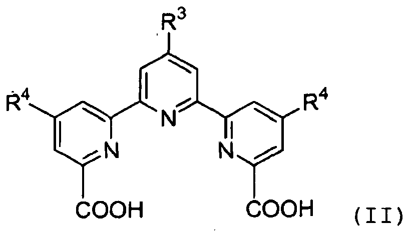 dans laquelle R³ et R⁴, identiques ou différents, ont la même signification que dans la formule générale (I), avec une amine de formule HNR¹R² dans laquelle R¹ et R², identiques ou différents, ont la même signification que dans la formule générale (I), en présence d'un agent de couplage peptidique et d'un catalyseur de couplage peptidique.

8. Procédé selon la revendication 7, dans lequel la réaction est réalisée en présence de chlorhydrate de 1-éthyl-3-[3-diméthylaminopropyl]-carbodiimide et de *N*-hydroxy-benzotriazole en solution dans un solvant organique dipolaire et aprotique.

9. Utilisation d'un composé selon l'une quelconque des revendications 1 à 6 en tant que ligand des actinides.

10. Utilisation selon la revendication 9, dans laquelle le composé est utilisé pour extraire un ou des actinides d'une solution aqueuse acide par la technique d'extraction liquide-liquide.

11. Utilisation selon la revendication 9, dans laquelle le composé est utilisé pour séparer les actinides présents dans une solution aqueuse acide des lanthanides qui sont également présents dans cette solution.

12. Utilisation selon la revendication 10 ou la revendication 11, dans laquelle la solution aqueuse acide est une solution d'acide nitrique de molarité au moins égale à 2.

13. Utilisation selon la revendication 12, dans laquelle la solution aqueuse acide est une solution issue de la dissolution d'un combustible nucléaire usé dans de l'acide nitrique.

14. Utilisation selon la revendication 12, dans laquelle la solution aqueuse acide est une solution issue de la dissolution d'un combustible nucléaire usé dans de l'acide nitrique, préalablement débarrassée de l'uranium qu'elle contient.

15. Utilisation selon l'une quelconque des revendications 10 à 14, dans laquelle le composé est utilisé en solution dans un solvant organique choisi parmi le *n*-octanol, le nitrobenzène, le *n*-dodécane et le tétrapropylène hydrogéné.

16. Utilisation selon l'une quelconque des revendications 11 à 15, qui comprend :
- l'extraction des actinides de la solution aqueuse par mise en contact avec une phase organique comprenant le composé dans un diluant organique puis séparation de ladite solution et de ladite phase ; et
- la désextraction des actinides présents dans la phase organique obtenue à l'issue de l'extraction par mise en contact de cette phase avec une phase aqueuse acide, de préférence de pH allant de 2 à 3.

## Patentansprüche

1. Verbindung, die der folgenden allgemeinen Formel (I) entspricht: 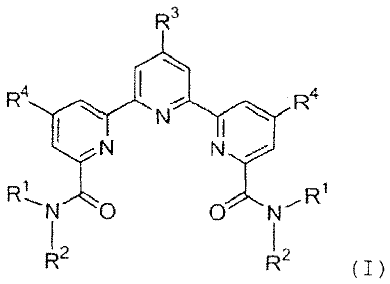 wobei:
R¹ und R², die identisch oder verschieden sind, ein Wasserstoffatom, eine gesättigte oder ungesättigte, lineare oder verzweigte Kohlenwasserstoffgruppe von C₁ bis C₁₂, eine Phenylgruppe, eine Benzylgruppe, eine Biphenylgruppe oder eine Tolylgruppe repräsentieren;
R³ ein Wasserstoffatom, eine gesättigte oder ungesättigte, lineare oder verzweigte Kohlenwasserstoffgruppe von C₁ bis C₁₂, eine Phenylgruppe, eine Tolylgruppe oder eine lineare oder verzweigte Alkoxygruppe von C₁ bis C₁₂ repräsentiert; hingegen
R⁴ ein Wasserstoffatom, eine gesättigte oder ungesättigte, lineare oder verzweigte Kohlenwasserstoffgruppe von C₁ bis C₁₂, eine Phenylgruppe oder eine Tolylgruppe repräsentiert.

2. Verbindung nach Anspruch 1, die der allgemeinen Formel (I) entspricht, wobei:
R¹ und R², die identisch oder verschieden sind, ein Wasserstoffatom, eine lineare oder verzweigte Alkylkette von C₁ bis C₁₂ oder eine Phenylgruppe repräsentieren,
R³ ein Wasserstoffatom oder eine lineare oder verzweigte Alkyl- oder Alkoxygruppe von C₁ bis C₁₂ repräsentiert; hingegen
R⁴ ein Wasserstoffatom oder eine lineare oder verzweigte Alkylgruppe von C₁ bis C₁₂ repräsentiert.

3. Verbindung nach Anspruch 1 oder Anspruch 2, die der allgemeinen Formel (I) entspricht, wobei R³ und R⁴ ein Wasserstoffatom repräsentieren.

4. Verbindung nach einem der vorhergehenden Ansprüche, die der allgemeinen Formel (I) entspricht, wobei, R¹ und R² zueinander identisch sind.

5. Verbindung nach Anspruch 4, die der allgemeinen Formel (I) entspricht, wobei R¹ und R² eine lineare oder verzweigte Alkylkette von C₁ bis C₁₂ und, stärker bevorzugt, von C₂, von C₄, von C₆, von C₈, von C₁₀ oder von C₁₂ repräsentieren.

6. Verbindung nach einem der vorhergehenden Ansprüche, die gewählt ist aus:
*N,N,N',N'*- Tetraethyl - 6, 6"- (2, 2' : 6', 2"- terpyridin) diamid;
*N,N,N',N'*- Tetrabutyl - 6, 6" - (2, 2': 6', 2" - terpyridin) diamid; und
*N,N,N',N'*- Tetraoctyl - 6, 6"- (2, 2': 6', 2"- terpyridin) diamid.

7. Verfahren zur Synthese einer Verbindung nach einem der Ansprüche 1 bis 6, das die Reaktion einer Verbindung der folgenden allgemeinen Formel (II) umfasst: 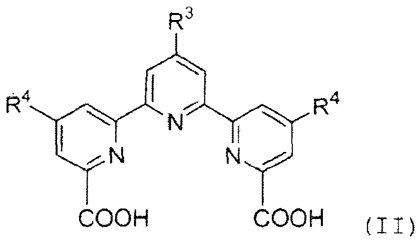 wobei R³ und R⁴, die identisch oder verschieden sind, die gleiche Bedeutung wie in der allgemeinen Formel (I) haben, und zwar mit einem Amin der Formel HNR¹R², in der R¹ und R², die identisch oder verschieden sind, die gleiche Bedeutung wie in der allgemeinen Formel (I) haben, bei Vorhandensein eines Peptidkupplers oder eines Peptidkupplungskatalysators.

8. Verfahren nach Anspruch 7, bei dem die Reaktion unter Vorhandensein von Chlorhydrat von 1 - Ethyl - 3 - [3 - dimethylaminopropyl] - carbodiimid und *N*- Hydroxy-benzotriazol in Lösung in einem organischen dipolaren und aprotischen Lösungsmittel durchgeführt wird.

9. Anwendung einer Verbindung nach einem der Ansprüche 1 bis 6 als Ligand von Actiniden.

10. Anwendung nach Anspruch 9, bei der die Verbindung verwendet wird, um eines oder mehrere Actinide einer wässrigen sauren Lösung durch ein Flüssig-Flüssig-Extraktionsverfahren zu extrahieren.

11. Anwendung nach Anspruch 9, bei der die Verbindung verwendet wird, um die Actinide zu trennen, die in einer wässrigen sauren Lösung von Lanthaniden vorhanden sind, welche ebenfalls in dieser Lösung vorhanden sind.

12. Anwendung nach Anspruch 10 oder Anspruch 11, bei der die wässrige saure Lösung eine Salpetersäurelösung ist, deren Molarität mindestens 2 beträgt.

13. Anwendung nach Anspruch 12, bei der die wässrige saure Lösung eine Lösung ist, die aus dem Auflösen eines verbrauchten Kernbrennstoffes in Salpetersäure hervorgeht.

14. Anwendung nach Anspruch 12, bei der die wässrige saure Lösung eine Lösung ist, die aus dem Auflösen eines verbrauchten Kernbrennstoffes in Salpetersäure hervorgeht und die zuvor von dem Uran, das sie enthält, befreit wurde.

15. Anwendung nach einem der Ansprüche 10 bis 14, bei der die Verbindung in Lösung in einem organischen Lösungsmittel verwendet wird, das gewählt ist aus *n*-Octanol, Nitrobenzol, n-Dodecan- und hydriertem Tetrapropylen.

16. Anwendung nach einem der Ansprüche 11 bis 15, die beinhaltet:
Extrahieren von Actiniden aus der wässrigen Lösung durch In-Kontakt-Bringen mit einer organischen Phase, welche die Verbindung in einem organischen Verdünnungsmittel enthält, und dann Trennen der Lösung und der Phase; und
Desextraktion der Actinide, die in der organischen Phase vorhanden sind, die am Ende der Extraktion erhalten wird, und zwar durch In-Kontakt-Bringen dieser Phase mit einer wässrigen sauren Phase, die vorzugsweise einen pH-Wert von 2 bis 3 hat.

## Claims

1. A compound which fits the general formula (I) hereafter: 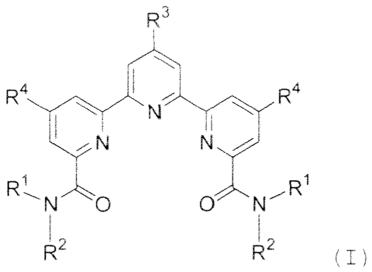 wherein:
- R¹ and R², either identical or different, represent a hydrogen atom, a linear or branched, saturated or unsaturated C₁-C₁₂ hydrocarbon group, a phenyl group, a benzyl group, a biphenyl group or a tolyl group;
- R³ represents a hydrogen atom, a linear or branched, saturated or unsaturated C₁-C₁₂ hydrocarbon group, a phenyl group, a tolyl group or a linear or branched C₁-C₁₂ alkoxy group; while
- R⁴ represents a hydrogen atom, a linear or branched, saturated or unsaturated C₁-C₁₂ hydrocarbon group, a phenyl group or a tolyl group.

2. The compound according to claim 1, which fits the general formula (I) wherein:
- R¹ and R², either identical or different, represent a hydrogen atom, a linear or branched C₁-C₁₂ alkyl chain or a phenyl group;
- R represents a hydrogen atom or a linear or branched C₁-C₁₂ alkyl or alkoxy group; while
- R⁴ represents a hydrogen atom or a linear or branched C₁-C₁₂ alkyl group.

3. The compound according to claim 1 or claim 2, which fits general formula (I) wherein R³ and R⁴ represent a hydrogen atom.

4. The compound according to any of the preceding claims, which fits general formula (I) wherein R¹ and R² are identical with each other.

5. The compound according to claim 4, which fits general formula (I) wheren R¹ and R² represent a linear or branched C₁ - C₁₂ and even better C₂, C₄, C₆, C₈, C₁₀, or C₁₂ alkyl chain.

6. The compound according to any of the preceding claims, which is selected from:
- *N,N,N',N'*-tetraethyl-6,6"-(2,2':6',2"-terpyridine)diamide,
- *N,N,N',N'*-tetrabutyl-6,6"-(2,2':6',2"-terpyridine)diamide; and
- *N,N,N',N'*-tetraoctyl-6,6"-(2,2':6',2"-terpyridine)diamide.

7. A method for synthesizing a compound according to any of claims 1 to 6, which comprises the reaction of a compound of general formula (II) hereafter: 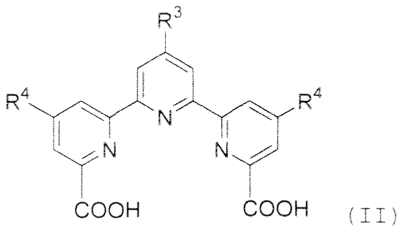 wherein R³ and R⁴, either identical or different have the same meaning as in the general formula (I), with an amine of formula HNR¹R², wherein R¹ and R², either identical or different have the same meaning as in the general formula (I), in the presence of a peptide coupling agent and a peptide coupling catalyst.

8. The method according to claim 7, wherein the reaction is conducted in the presence of 1-ethyl-3-[3-dimethylaminopropyl]-carbodiimide hydrochloride and *N*-hydroxy-benzotriazole in solution in a dipolar and aprotic organic solvent.

9. The use of a compound according to any of claims 1 to 6 as a ligand of actinides.

10. The use according to claim 9, wherein the compound is used for extracting actinide(s) from an acid aqueous solution by the liquid-liquid extraction technique.

11. The use according to claim 9, wherein the compound is used for separating the actinides present in an acid aqueous solution from the lanthanides which are also present in this solution.

12. The use according to claim 10 or claim 11, wherein the acid aqueous solution is a nitric acid solution with a molarity at least equal to 2.

13. The use according to claim 12, wherein the acid aqueous solution is a solution stemming from the dissolution of a used nuclear fuel in nitric acid.

14. The use according to claim 12, wherein the acid aqueous solution is a solution stemming from the dissolution of a used nuclear fuel in nitric acid, after having cleared it of the uranium which it contains.

15. The use according to any of claims 10 to 14, wherein the compound is used in solution in an organic solvent selected from n-octanol, nitrobenzene, n-dodecane and hydrogenated tetrapropylene.

16. The use according to any of claims 11 to 15, which comprises:
- extraction of the actinides from the aqueous solution by putting it into contact with an organic phase comprising the compound in an organic diluent and then separation of said solution and of said phase; and
- de-extraction of the actinides present in the organic phase obtained at the end of the extraction by putting this phase into contact with an acid aqueous phase, preferably with a pH ranging from 2 to 3.
